# EUROPEAN PATENT APPLICATION

(11) **EP 1 559 419 A1**
(43) Date of publication of application: **03.08.2005**
(21) Application number: 04001499.5
(22) Date of filing: 23.01.2004
(51) Int. Cl.: A61K 9/16, A61K 31/216, A61K 31/155

(54) **Pharmaceutical formulations comprising metformin and a fibrate, and processes for their obtention**

(71) Applicant: Fournier Laboratories Ireland Limited, Carrigtwohill, Co. Cork (IE)
(72) Inventor: Dawson, Gordon, Tivoli, Co. Cork (IE); McCarthy, Leonard, Glanmire, Co.Cork (IE)
(74) Representative: Nevant, Marc

(57) **Abstract**

The present invention relates to granulates comprising particles of metformin surrounded by particles of a fibrate. The invention further relates to pharmaceutical compsitions containing such granulates. The invention also relates to processes for preparing said granulates and said pharmaceutical compositions.

## Description

The present invention relates to granulates comprising metformin and a fibrate, such as fenofibrate, to pharmaceutical compositions containing such granulates, and to processes for preparing said granulates and said composition.

### Background of the invention

Combination products present several challenges to the pharmaceutical scientist beyond those that are inherent with the development of any pharmaceutical product. These additional challenges arise for several reasons, including: a requirement to ensure that the combination is stable, a requirement to ensure that the dosage form produced is acceptable to patients despite potentially large doses of the individual components and a requirement to match the pharmacokinetic performance of each active in the combination to that resulting from administration of the drugs as monotherapy. The latter requirement is especially important to ensure combination product safety and efficacy. These challenges are greater if the physical chemistry of the active compounds being combined is significantly different. For example, if it is required to combine a hydrophobic active with a hydrophilic active or a water soluble active with a water insoluble active the challenge to the formulator is expected to be great. In addition to these general considerations, the combination must encompass formulation strategies that address specific pharmaceutical problems associated with each of the actives. For example, it may be found that one active does not compress well, thus restricting the choice of binder or compression aid. Alternatively, it may be found that the flow properties of one active are not conducive to high output manufacturing thus dictating a choice of glidant, lubricant or other external phase components.

Combinations of hydrophobic, water insoluble active compounds with hydrophilic, water-soluble compounds are a particular challenge if the pharmacokinetic properties of either or both of the actives are known to be affected by the formulation.

Metformin is a biguanide that is mainly known for its antihyperglycaemic activity and is widely used in the treatment of non-insulin dependent diabetes; metformin can also be administered to the patient in combination with insulin.

Metformin is freely soluble in water (Martindale, 33^{rd} Ed, pp332 (2002)). It is also known to be a poorly compressible substance. A poorly compressible substance is one that does not bind to form a tablet upon application of compression force. Therefore, such substances may require additional processing and special formulating before they can be compressed into tablets. With such substances, the additional processing necessary is usually a wet granulation step, as direct compression would not be effective. These substances may be formulated with binders or other materials that have high binding capacity (or that act as an aid to compressibility) such that the non-bonding properties of the non-compressible material are overcome. Other techniques to assist compression include having residual moisture in the blend prior to compression or having the non-compressible material in very low amounts in the tablet formulation. High-dose drugs, such as metformin, do not lend themselves to direct compression because of the relatively low proportion of diluent or compression aid in the tablet, poor powder flow and poor compressibility.

Fibrates are known for their anti-hyperlipidic properties. More specifically, the fibrates act on hypercholesterolaemia by inducing a reduction in the total cholesterol level as well as the cholesterol linked to low density lipoproteins (LDL-cholesterol) and an even greater reduction in the level of triglycerides and in particular of triglycerides linked to very low density lipoproteins (VLDL-triglycerides).

Generally, fibrates are used for conditions such as hypercholesterolemia, mixed lipidemia, hypertriglyceridemia, coronary heart disease, and peripheral vascular disease (including symptomatic carotid artery disease), and prevention of pancreatitis. Fenofibrate may also help prevent the development of pancreatitis (inflammation of the pancreas) caused by high levels of triglycerides in the blood. Fibrates are known to be useful in treating renal failure (U.S. Patent No. 4,250,191). Fibrates may also be used for other indications where lipid regulating agents are typically used.

Fenofibrate, also known as 2-[4-(4-chlorobenzoyl) phenoxy]-2-methyl-propanoic acid, 1-methylethyl ester, is a lipid regulating agent. The compound is insoluble in water. *See The Physicians' Desk Reference*, 56^{th} Ed., pp. 513-516 (2002) and Martindale, 33^{rd} Ed, pp889 (2002).

Fenofibrate is described in, for example, U.S. Patent Nos. 3,907,792 for "Phenoxy-Alkyl-Carboxylic Acid Derivatives and the Preparation Thereof"; 4,895,726 for "Novel Dosage Form of Fenofibrate"; 6,074,670 and 6,277,405, both for "Fenofibrate Pharmaceutical Composition Having High Bioavailability and Method for Preparing It". U.S. Patent No. 3,907,792 describes a class of phenoxy-alkyl carboxylic compounds which encompasses fenofibrate.

A variety of clinical studies have demonstrated that elevated levels of total cholesterol (total-C), low density lipoprotein cholesterol (LDL-C), and apolipoprotein B (apo B), an LDL membrane complex, are associated with human atherosclerosis. Similarly, decreased levels of high density lipoprotein cholesterol (HDL-C) and its transport complex, apolipoprotein A (apo A2 and apo AII), are associated with the development of atherosclerosis. Epidemiologic investigations have established that cardiovascular morbidity and mortality vary directly with the level of total-C, LDL-C, and triglycerides, and inversely with the level of HDL-C.

Fenofibric acid, the active metabolite of fenofibrate, produces reductions in total cholesterol, LDL cholesterol, apo-lipoprotein B, total triglycerides, and triglyceride rich lipoprotein (VLDL) in treated patients. In addition, treatment with fenofibrate results in increases in high density lipoprotein (HDL) and apolipoprotein apoAI and apoAII. *See The Physicians' Desk Reference,* 56^{th} Ed., pp. 513-516 (2002).

Fenofibrate is used to lower triglyceride (fat-like substances) levels in the blood. Specifically, fenofibrate reduces elevated LDL-C, Total-C, triglycerides, and Apo-B and increases HDL-C. The drug has also been approved as adjunctive therapy for the treatment of hypertriglyceridemia, a disorder characterized by elevated levels of very low density lipoprotein (VLDL) in the plasma.

The mechanism of action of fenofibrate has not been clearly established in man. Fenofibric acid, the active metabolite of fenofibrate, lowers plasma triglycerides apparently by inhibiting triglyceride synthesis, resulting in a reduction of VLDL released into the circulation, and also by stimulating the catabolism of triglyceride-rich lipoprotein (*i.e.*, VLDL). Fenofibrate also reduces serum uric acid levels in hyperuricemic and normal individuals by increasing the urinary excretion of uric acid.

Fenofibrate is a compound that is associated with a low bioavailability following oral administration, a property that has been attributed to its low solubility and hydrophobic character. Furthermore, the bioavailability of fenofibrate is substantially lower when it is administered to fasted patients compared to fed patients. Considerable effort has been expended to develop pharmaceutical forms of fenofibrate with improved bioavailability and less variability between the fed and fasted states. For example, U.S. Patent No. 4,895,726 describes a gelatin capsule therapeutic composition, useful in the oral treatment of hyperlipidemia and hypercholesterolemia, containing micronized fenofibrate. U.S. Patent No. 6,074,670 refers to immediate-release fenofibrate compositions comprising micronized fenofibrate and at least one inert hydrosoluble carrier. U.S. Patent No. 6,277,405 is directed to micronized fenofibrate compositions having a specified dissolution profile. In addition, WO 02/24192 for "Stabilised Fibrate Microparticles", describes a microparticulate fenofibrate composition comprising a phospholipid. WO 02/067901 for "Fibrate-Statin Combinations with Reduced Fed-Fasted Effects", describes a microparticulate fenofibrate composition comprising a phospholipid and a hydroxymethylglutaryl coenzyme A (HMG-CoA) reductase inhibitor or statin. WO 03/013474 for "Nanoparticulate Formulations of Fenofibrate", describes fibrate compositions comprising vitamin E TGPS (polyethylene glycol (PEG) derivative vitamin E).

EP 1 054 665 discloses a combination of metformin and of a fibrate chosen from fenofibrate and bezafibrate for the treatment of non-insulin-dependent diabetes. In this patent application, no specific formulation and no specific method for producing a pharmaceutical formulation of a fibrate and metformin are disclosed.

### Summary of the invention

An objective of the invention is to provide a composition that addresses the general challenges associated with the development of a pharmaceutical product, the specific challenges associated with the individual active compounds incorporated in the dosage form and also the challenges associated with bringing the active substances into combination. A particular challenge associated with this combination is to ensure the bioequivalence of each active compound to the respective components when administered separately in spite of the biopharmaceutical problems associated with fenofibrate and the different physical and chemical properties of both actives.

Another objective of the invention is to obtain a formulation of a fibrate and metformin with a size suitable for administration and acceptable to patients in spite of the fact that the composition of the invention shall contain a high amount of metformin (metformin is usually prescribed at 850 mg once or twice a day or at 500 mg, three to four times a day). This considerable mass of metformin is to be combined in the same pharmaceutical dosage unit with a fibrate, which, although as an active compound is required in smaller quantities than metformin (for example, fenofibrate is prescribed once daily at 200 mg or at 160 mg for Tricor 160®), is taught by the prior art as being associated with a large quantity of excipient in order to maintain an acceptable bio-availability (as taught by in U.S. Patent No. 6,074,670).

A further objective of the invention is to obtain a formulation which gives rise to high patient compliance, by reducing the number of unit forms of administration, such as tablets, that need to be taken. Diabetes mellitus type II often requires treatment with more than one active substance. In addition, amongst type II diabetes, the prevalence of other disorders associated with insulin resistance (dyslipidaemia, hypertension), which frequently require additional pharmacological forms of treatment, is high. Patient compliance under such circumstances is quite a problem, because individual dosage units are necessarily quite large in view of the high amounts of active substances which need to be administered (as discussed above), and the practical limits as regards the mass of pharmaceutical compositions which can be administered to a patient as a single dosage unit.

A further objective of the invention is to avoid a possible interaction between the fibrate and metformin, which could impair the bioavailability of the fibrate and/or metformin.

Metformin being a hydrophilic component and the fibrate a hydrophobic component, the biodisponibility of both active components can be hindered, in a single composition. The presence of a hydrophobic fibrate can indeed impair the biodisponibility of metformin by retarding its dissolution with a consequent effect on bioavailability. Because fibrates, including fenofibrate, are so insoluble in water, significant bioavailability can be problematic.

Thus, a further objective of this invention is to provide a composition containing both active components, metformin and a fibrate, whilst maintaining a bioavailability of both components equivalent to that obtained with metformin alone or with the fibrate alone.

Another objective of the invention is to provide processes for preparing the pharmaceutical compositions fulfilling the objectives listed above, such processes being able to be accomplished with a limited number of different steps and being inexpensive.

It has now unexpectedly be found that granulates comprising particles of metformin surrounded by particles of a fibrate, make it possible to prepare pharmaceutical compositions enabling the above-cited objectives to be attained.

Thus, the invention provides granulates which comprise particles of metformin which are either isolated or agglomerated, and particles of a fibrate, adhering to said metformin particles.

Preferably, the granulates of the present invention consist of metformin particles, to which particles of fenofibrate adhere. The particles of the fibrate, preferably fenofibrate, and the particles of metformin can be bound together with a suitable binder. The average particle size of the fibrate particles will preferably be smaller than that of the metformin particles.

The invention further provides pharmaceutical compositions comprising granulates as defined above.

The invention also provides processes for the manufacture of the said granulates.

In addition, the invention provides a process for the manufacture of pharmaceutical compositions containing such granulates.

According to the invention, a reduced amount of excipients can be used in the preparation of the pharmaceutical compositions, which further have a size suitable for administration whilst maintaining bio-equivalence to monotherapy i.e. separate administration of metformin and a fibrate.

Furthermore, it is possible to maintain the same bioavailability as is obtained when each component is administered separately, whilst nonetheless using dosage units which have an acceptable size from the point of view of administration to the patient.

The compositions of the invention further enable an improvement in patient convenience with a reduction in the number of tablets that need to be taken, thereby increasing subject compliance.

This is significant, as with poor subject compliance a deterioration of the medical condition for which the drug is being prescribed may be observed, *i.e.*, cardiovascular problems for poor subject compliance treated with a fibrate or with metformin.

The pharmaceutical compositions of the present invention can be used for the treatment of non-insulin dependent diabetes mellitus (or type 2 diabetes), dyslipidemia with impaired glucose tolerance, hyperlipidemia, hypercholesterolemia, for the prevention of cardiovascular events, for the treatment and prevention of metabolic syndrome and for the treatment or prevention of any illness in which a treatment with a fibrate and metformin is desirable, such as obesity.

The invention will be described in more detail in the description which follows, with reference to the attached drawings.

### Brief description of drawings

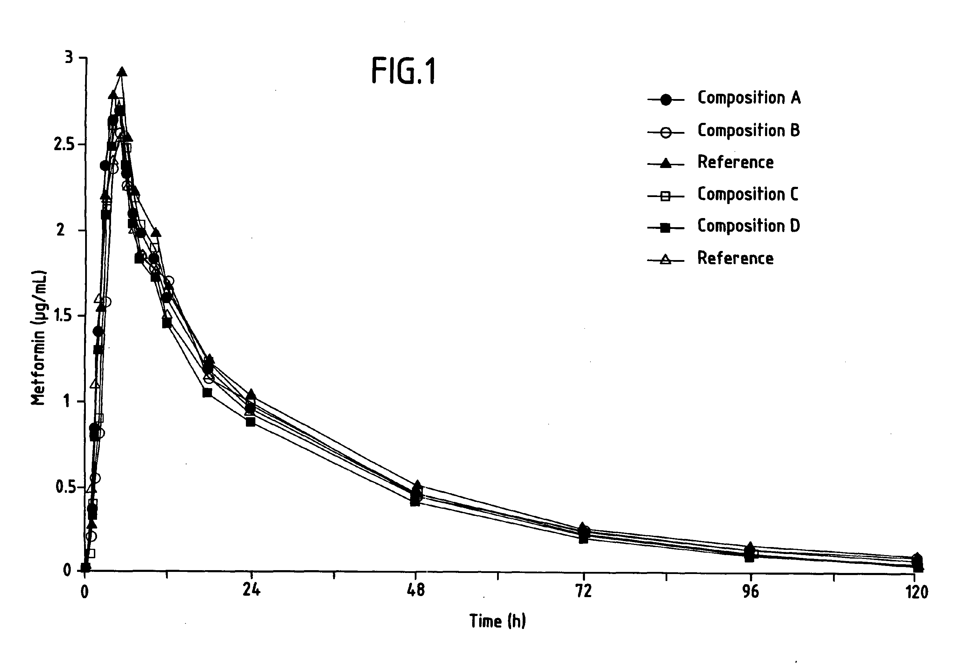

Figure 1 shows the bioequivalence between the test product according to the present invention and 80 mg fenofibrate reference therapy.

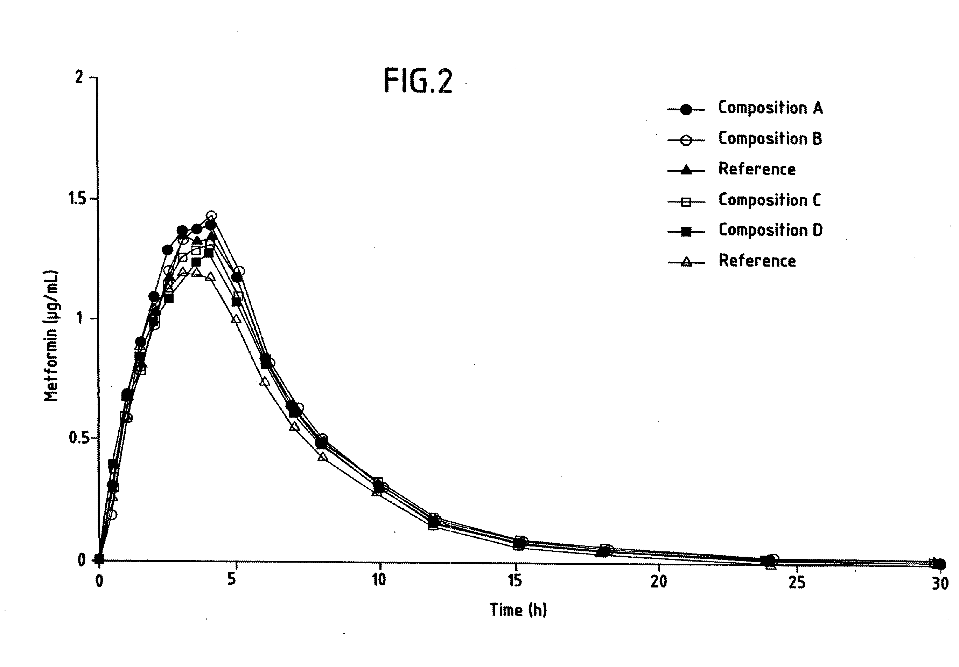

Figure 2 shows the bioequivalence between the test product according to the invention and 850 mg of metformin reference therapy.

### Detailed description of preferred embodiments

The granulates according to the present invention comprise particles of metformin, which are either isolated or agglomerated, and particles of a fibrate adhering to said metformin particles.

Advantageously, the fibrate is in a crystalline phase, an amorphous phase, a semi-crystalline phase, or a semi-amorphous phase.

The fibrate can be selected from the group consisting of gemfibrozil, fenofibrate, bezafibrate, clofibrate, ciprofibrate, beclobrate, binifibrate, ciplofibrate, clinofibrate, etofibrate, nicofibrate, pirifibrate, ronifibrate, simfibrate, theofibrate, a fibric acid derivative (e.g. fenofibric acid or clofibric acid) or a pharmaceutically acceptable salt or ester of said fibric acid derivative.

Preferably, the fibrate is fenofibrate, fenofibric acid or a pharmaceutically acceptable salt or ester of fenofibric acid.

In a preferred embodiment of the invention, the fibrate is fenofibrate. As used herein the term fenofibrate, is used to mean (2-[4-(4-chlorobenzoyl) phenoxy]-2-methyl-propanoic acid, 1-methylethyl ester) or a salt thereof.

The fibrate can be of a reduced particle size. The fibrate can for example be micronised or co-micronised with a surfactant. Any surfactant is suitable, whether it be amphoteric, non-ionic, cationic or anionic. Examples of such surfactants are: sodium lauryl sulfate, monooleate, monolaurate, monopalmitate, monostearate or another ester of polyoxyethylene sorbitane, sodium dioctylsulfosuccinate (DOSS; also known as sodium docusate), lecithin, stearylic alcohol, cetostearylic alcohol, cholesterol, polyoxyethylene ricin oil, polyoxyethylene fatty acid glycerides, a poloxamer and mixtures thereofalso suitable. The preferred surfactant is sodium lauryl sulfate, which can be (co-) micronized with fenofibrate such as described in EP 0 330 532.

In one preferred embodiment of the invention, the fibrate particles have an average particle size of less than about 20 µm, preferably of less than about 10 µm.

The fibrate can also be in the form of nanoparticles which can be obtained using, for example, milling, homogenization, or precipitation techniques. Methods of making nanoparticulate compositions are also described in U.S. Patent No. 5,518,187 for "Method of Grinding Pharmaceutical Substances"; U.S. Patent No. 5,718,388 for "Continuous Method of Grinding Pharmaceutical Substances"; U.S. Patent No. 5,862,999 for "Method of Grinding Pharmaceutical Substances"; U.S. Patent No. 5,665,331 for "Co-Microprecipitation of Nanoparticulate Pharmaceutical Agents with Crystal Growth Modifiers"; U.S. Patent No. 5,662,883 for "Co-Microprecipitation of Nanoparticulate Pharmaceutical Agents with Crystal Growth Modifiers"; U.S. Patent No. 5,560,932 for "Microprecipitation of Nanoparticulate Pharmaceutical Agents"; U.S. Patent No. 5,543,133 for "Process of Preparing X-Ray Contrast Compositions Containing Nanoparticles"; U.S. Patent No. 5,534,270 for "Method of Preparing Stable Drug Nanoparticles"; U.S. Patent No. 5,510,118 for "Process of Preparing Therapeutic Compositions Containing Nanoparticles"; U.S. Patent No. 5,470,583 for "Method of Preparing Nanoparticle Compositions Containing Charged Phospholipids to Reduce Aggregation"; and US patent application 2003/0 224 058 for "Nanoparticulate fibrate formulations", all of which are specifically incorporated by reference.

Nanoparticulate fibrate dispersions can be obtained by milling: for example, milling a fibrate, preferably fenofibrate, to obtain a nanoparticulate dispersion comprises dispersing the fibrate particles in a liquid dispersion medium in which the fibrate is poorly soluble, followed by applying mechanical means in the presence of grinding media to reduce the particle size of the fibrate to the desired effective average particle size. The dispersion medium can be, for example, water, sunflower oil, ethanol, t-butanol, glycerin, polyethylene glycol (PEG), hexane, or glycol. A preferred dispersion medium is water.

The fibrate, preferably fenofibrate, particles can be reduced in size in the presence of at least one surface stabilizer. Alternatively, the fibrate particles can be contacted with one or more surface stabilizers after attrition. Other compounds, such as a diluent, can be added to the fibrate/surface stabilizer composition during the size reduction process. Dispersions can be manufactured continuously or in a batch mode. Combinations of more than one surface stabilizer can be used. Useful surface stabilizers which can be employed include, but are not limited to, known organic and inorganic pharmaceutical excipients. Such excipients include various polymers, low molecular weight oligomers, natural products, and surfactants. Surface stabilizers include nonionic, anionic, cationic, ionic, and zwitterionic surfactants. Suitable surface stabilizers are those mentioned in US patent application 2003/0224058, the contents of which are incorporarted herein by reference, and described below.

Another method of forming the desired nanoparticulate fibrate, preferably fenofibrate, is by microprecipitation. This is a method of preparing stable dispersions of poorly soluble active agents in the presence of one or more surface stabilisers and one or more colloid stability enhancing surface active agents free of any trace of toxic solvents or of solubilized heavy metal impurities. Such a method comprises, for example: (1) dissolving the fibrate in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means.

Nanoparticulate fibrate can also be obtained by homogenization: exemplary homogenisation methods of preparing active agent nanoparticulate compositions are described in U.S. Patent No. 5,510,118, for "Process of Preparing Therapeutic Compositions Containing Nanoparticles". Such a method comprises dispersing particles of a fibrate, preferably fenofibrate, in a liquid dispersion medium, followed by subjecting the dispersion to homogenisation to reduce the particle size of the fibrate to the desired particle size. The fibrate particles can be reduced in size in the presence of at least one surface stabilizer. Alternatively, the fibrate particles can be contacted with one or more surface stabilizers either before or after attrition. Other compounds, such as a diluent, can be added to the fenofibrate/surface stabilizer composition either before, during, or after the size reduction process. Dispersions can be manufactured continuously or in a batch mode.

According to the invention, the nanoparticulate fibrate has an average particle size of less than about 2000 nm, e.g. less than about 1900 nm, less than about 1800 nm, less than about 1700 nm, less than about 1600 nm; preferably less than about 1500 nm, e.g. less than about 1400 nm, less than about 1300 nm, less than about 1200 nm, less than about 1100 nm; preferably less than about 1000 nm, e.g. less than about 900 nm, less than about 800 nm, less than about 700 nm, less than about 600 nm; preferably less than about 500 nm, e.g. less than about 400 nm, less than about 300 nm, less than about 250 nm, less than about 200 nm; preferably less than about 100 nm, e.g. less than about 75 nm, or less than about 50 nm.

When preparing the granulates of the invention, metformin can be used either as the free base or in the form of a pharmaceutically acceptable acid addition salt thereof such as the hydrochloride, acetate, benzoate, citrate, fumarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octonoate, decanoate, hexadecanoate, octodecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate or phosphate.

Among these salts, the hydrochloride, fumarate, embonate and chlorophenoxyacetate are more particularly preferred, the hydrochloride being especially preferred.

Advantageously, the metformin particles have an average size in the range of between 50 µm and 500 µm.

According to an embodiment of the invention the particles of metformin and of fibrate are bound with a suitable binder as defined below for the pharmaceutical compositions of the invention. The amount of binder used is generally in the range of from about 0.1 to about 40 wt %, preferably about 0.1 to about 20 wt %, and more preferably about 0.1 to about 10 wt %, of the total amount of metformin and the fibrate.

The granulates according to the invention are useful in the preparation of pharmaceutical compositions; such compositions may comprise one or more excipients known in the art. Such excipients include (a) surface stabilizers, (b) binders, (c) filling agents, (d) lubricating agents, (e) glidants, (f) suspending agents, (g) sweeteners, (h) flavoring agents, (i) preservatives, (j) buffers, (k) wetting agents, (l) disintegrants, (m) effervescent agents, (n) humectants, (o) solution retarders, (p) absorption accelerators, (q) adsorbents.

### a) Surface stabilizers

Examples of surface stabilizers which may be used within the framework of of the invention are polymers, low molecular weight oligomers, natural products, and surfactants, including nonionic, anionic, cationic, ionic, and zwitterionic surfactants, as well as mixtures thereof.

Representative examples of surface stabilizers include hydroxypropyl methylcellulose (now known as hypromellose), hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters (e.g. the commercially available Spans® such as Span® 80 and Span® 20), polyoxyethylene alkyl ethers (e.g., macrogol ethers such as cetomacrogol 1000), polyoxyethylene castor oil derivatives (polyoxols) (e.g. the commercially available Cremophors®), polyoxyethylene sorbitan fatty acid esters (e.g., the commercially available Tweens® such as e.g., Tween 20® and Tween 80® (ICI Speciality Chemicals)); polyethylene glycols (e.g., Carbowaxs 3550® and 934® (Union Carbide)), polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as Tyloxapol®, Superione®, and Triton®), poloxamers (e.g., Pluronics F68® and F108®, which are block copolymers of ethylene oxide and propylene oxide); poloxamines (e.g., Tetronic 908®, also known as Poloxamine 908®, which is a tetrafunctional block copolymer derived from sequential addition of propylene oxide and ethylene oxide to ethylenediamine (BASF Wyandotte Corporation, Parsippany, N.J.)); Tetronic 1508® (T-1508) (BASF Wyandotte Corporation); Triton X-200®, which is an alkyl aryl polyether sulfonate (Rohm and Haas); Crodestas F-110®, which is a mixture of sucrose stearate and sucrose distearate (Croda Inc.); p-isononylphenoxypoly-(glycidol), also known as Olin-10G® or Surfactant 10-G® (Olin Chemicals, Stamford, Conn.); Crodestas SL-40® (Croda, Inc.); and SA9OHCO® (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl β-D-glucopyranoside; n-heptyl β-D-thioglucoside; n-hexyl β-D-glucopyranoside; nonanoyl-N-methylglucamide; n-nonyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

If desirable, the nanoparticulate fibrate, preferable fenofibrate, compositions of the invention can be formulated to be phospholipid-free.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, alkyl-dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, polydiallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT® 336), POLYQUAT® 10, tetrabutylammonium bromide, benzyl trimethylammonium bromide, choline esters (such as choline esters of fatty acids), benzalkonium chloride, stearalkonium chloride compounds (such as stearyltrimonium chloride and di-stearyldimonium chloride), cetyl pyridinium bromide or chloride, halide salts of quaternized polyoxyethylalkylamines, MIRAPOL® and ALKAQUAT® (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Particularly preferred surface stabilizers to be used within the framework of the present invention are sodium lauryl sulfate, sodium sulfosuccinate (sodium docusate, DOSS) and polyoxyethylene sorbitan fatty acid esters.

### b) Binders

Examples of binders to be used within the framework of the present invention include acacia gum, alginic acid, carboxymethylcellulose, carboxymethylcellulose sodium, carboxyethylcellulose, dextrin, ethylcellulose, gelatin, guar gum, hydrogenated vegetable oil, hydroxyethylcellulose, hydroxypropylcellulose, hydroxypropylmethylcellulose, liquid glucose, magnesium aluminium silicate, maltodextrin, methylcellulose, polymethacrylates, povidone, pre-gelatinised starch, sodium alginate, starch, xanthan gum, tragacanth gum, zein and mixtures thereof.

In a particularly preferred embodiment of the invention, the binder is selected from povidone and / or hydroxypropyl(methyl)cellulose.

### c) Filling agents

Examples of filling agents which may be used within the framework of the present invention include calcium carbonate, calcium sulphate, sucrose, dextrates, dextrin, calcium phosphate, glyceryl palmitostearate, hydrogenated vegetable oil, kaolin, lactose, magnesium carbonate, magnesium oxide, maltodextrin, mannitol, microcrystalline cellulose, silicified microcrystalline cellulose, polymethacrylates, potassium chloride, powdered cellulose, pre-gelatinised starch, sodium chloride, sorbitol, starch, talc and calcium phosphate and mixtures thereof.

### d) Lubricating agents

Examples of lubricating agents (lubricants) which may be used within the framework of the present invention include calcium stearate, glyceryl monostearate glyceryl palmitostearate, hydrogenated castor oil, hydrogenated vegetable oil, light mineral oil, magnesium stearate, mineral oil, polyethylene glycol, sodium benzoate, sodium lauryl sulphate, sodium stearyl fumarate, stearic acid, talc and zinc stearate and mixtures thereof.

### e) Glidants

Examples of glidants which may be used within the framework of the present invention include colloidal silicon dioxide, magnesium trisilicate, powdered cellulose, starch, talc and calcium phosphate and mixtures thereof.

### f) Suspending agents

Examples of suspending agents which may be used within the framework of the present invention include acacia, agar, carageenan, guar gum, sodium alginate, starch, tragacanth, xanthan gum, carmellose sodium, hydroxyethylcellulose, hydroxypropylcellulose, hydroxypropylmethylcellulose, methylcellulose, microcrystalline cellulose, dispersible cellulose, propylene glycol alginate, aluminum magnesium silicate, bentonite, carbomers, colloidal anhydrous silica, polyvinyl alcohol, povidone, and gelatin.

### g) Sweeteners

Examples of sweeteners which may be used within the framework of the present invention include any natural or artificial sweetener, such as sucrose, dextrose, glycerin, lactose, liquid glucose, mannitol, sorbitol, xylitol, acesulfame potassium, aspartame, saccharin, saccharin sodium, sodium cyclamate and mixtures thereof.

### h) Flavoring agents

Examples of flavoring agents which may be used within the framework of the present invention include ethyl maltol, ethyl vanillin, fumaric acid, malic acid, maltol, menthol, vanillin, Magnasweet® (trademark of MAFCO), bubble gum flavor, and fruit flavors, and the like.

### i) Preservatives

Examples of preservatives which may be used within the framework of the present invention include alcohol, benzalkonium chloride, benzethonium chloride, benzoic acid, benzyl alcohol, bronopol, butyl-paraben, cetrimide, chlorhexidine, chlorobutanol, chlorocresol, cresol, ethylparaben, glycerin, imidurea, methylparaben, phenol, phenoxyethanol, phenylethyl alcohol, phenyl mercuric acetate, phenyl mercuric borate, phenylmercuric nitrate, potassium sorbate, propylene glycol, propyl-paraben, sodium benzoate, sodium propionate, sorbic acid and thiomersal.

### (j) Buffers

Examples of buffers which may be used within the framework of the present invention include phosphate, bicarbonate, tris-hydroxymethylethylamine, glycine, borate, citrate.

### (k) Wetting agents

Examples of wetting agents which may be used within the framework of the present invention include cetyl alcohol, sodium lauryl sulphate, poloxamers, polyoxethylene sorbitan fatty acid derivatives, glycerol monostearate.

### (l) Disintegrants

Examples of disintegrants which may be used within the framework of the present invention include alginic acid, carboxymethylcellulose calcium, carboxymethylcellulose sodium, colloidal silicon dioxide, croscarmellose sodium, crospovidone, guar gum, magnesium aluminium silicate, methylcellulose, microcrystalline cellulose, polacrilin sodium, powdered cellulose, pre-gelatinised starch, sodium alginate, sodium starch glycolate, starch, and mixtures thereof.

### (m) Effervescent agents

Examples of effervescent agents which may be used within the framework of the present invention include effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### (n) Humectants

As an example of humectant which can be used within the framework of the present invention, glycerol may be mentioned.

### (o) Solution retarders

As an example of solution retarder which can be used within the framework of the present invention, paraffin may be mentioned.

### (p) Absorption accelerators

As examples of absorption accelerators which can be used within the framework of the present invention, quaternary ammonium compounds may be mentioned.

### (q) Adsorbents

As examples of adsorbents which can be used within the framework of the present invention, kaolin and bentonite may be mentioned.

In one embodiment of the invention, the excipients of the invention can be selected from the group consisting of hydroxypropylmethylcellulose, lactose, croscarmellose sodium, (cros)povidone, guar and xanthan gums, polyethylene glycol, (microcrystalline) cellulose, hydroxypropylcellulose, hydroxyethylcellulose, carboxymethylcellulose, carboxyethylcellulose, sodium carboxymethylcellulose, sodium alginate, methyl cellulose, acacia gum, tragacanth gum, polyethylene oxide, magnesium stearate, colloidal silicon dioxide and mixtures thereof.

Any other binder that enables an improvement in the compressibility of metformin can also be used, such as for example pregelatinised starch, glucose or sucrose.

The compositions of the invention can be administered to a subject via any conventional means including, but not limited to, orally, rectally, ocularly, parenterally (*e.g.*, intravenous, intramuscular, or subcutaneous), intracisternally, pulmonary, intravaginally, intraperitoneally, locally (*e.g.*, powders, ointments or drops), or as a buccal or nasal spray. As used herein, the term "subject" is used to mean an animal, preferably a mammal, including a human or non-human. The terms patient and subject may be used interchangeably.

A preferred dosage form of the invention is a solid dosage form, although any pharmaceutically acceptable dosage form can be utilized. Exemplary solid dosage forms include, but are not limited to, tablets, capsules, sachets, lozenges, powders, pills, or granules, and the solid dosage form can be, for example, a fast melt dosage form, chewable form, controlled release dosage form, lyophilized dosage form, delayed release dosage form, extended release dosage form, pulsatile release dosage form, mixed immediate release and controlled release dosage form, or a combination thereof. A solid dosage form, such as a tablet or a capsule, is preferred.

Liquid dosage forms for oral administration include pharmaceutically acceptable emulsions, solutions, suspensions, syrups, and elixirs. The liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like. Besides such inert diluents, the composition can also include adjuvants, such as wetting agents, emulsifying and suspending agents, sweetening, flavoring, and perfuming agents.

The effective amount of fibrate and metformin in the compositions of the invention will be a glycaemic or lipidaemic disorder or disease suppressing treatment or prevention effective amount.

As used herein, an "effective amount" means the dose or effective amount to be administered to a patient and the frequency of administration to the subject can be readily determined by one of ordinary skill in the art by the use of known techniques and by observing results obtained under analogous circumstances. The dose or effective amount to be administered to a patient and the frequency of administration to the subject can be readily determined by one of ordinary skill in the art by the use of known techniques and by observing results obtained under analogous circumstances. In determining the effective amount or dose, a number of factors are considered by the attending diagnostician, including but not limited to, the potency and duration of action of the compounds used; the nature and severity of the illness to be treated as well as the sex, age weight, general health and individual responsiveness of the patient to be treated, and other relevant circumstances.

In one embodiment of the invention, the amount of the fibrate, can be chosen between 10 to 3000 mg.

In another embodiment of the invention, when the fibrate is fenofibrate, the amount of fenofibrate is preferably chosen from about 10 to 300 mg, more preferably from 40 to 90 mg.

In a further embodiment, when the fibrate is fenofibrate, the composition of the invention contains a daily dose of fenofibrate, such as a 160 mg fenofibrate dose as used in Tricor®160, or a dose bioequivalent to such a Tricor®160 formulation.

The amount of metformin or one of its pharmaceutically acceptable salts can be chosen in an amount ranging from about 100 mg to 3000 mg, and more preferably from about 200 mg to 2000 mg.

The relative amounts of a fibrate, preferably fenofibrate, and metformin can vary widely. The weight ratio of the fibrate to metformin can vary from 1:0.2 to 1:200. The optimal amount of the individual components can depend, for example, upon the particular fibrate selected. In one embodiment of the invention, it is preferred that the weight ratio of the amount of fibrate to the amount of metformin that is administered to the subject is within a range of from about 1:2, preferably 1:5 to 1:40 and more preferably 1:5 to 1:20.

The ratio of fibrate to metformin in the composition of the invention will vary depending on whether the present pharmaceutical composition is to be taken more than once a day, or is to be only taken once a day.

For a once-a-day form, the weight ratio of fenofibrate to metformin will be from 1:0.2 to 1:200, preferably 1:2 to 1:50, more preferably 1:8 to 1:25. For a twice-a-day form, the weight ratio of fenofibrate to metformin will be from 1:0.2 to 1:200, preferably 1:2 to 1:50, more preferably 1:5 to 1:12. For a three-times-a-day form, the ratio of fenofibrate to metformin will be from 1:0.2 to 1:200, preferably 1:2 to 1:20, more preferably 1:8 to 1:20. For a four-times-a-day form, the ratio of fenofibrate to metformin will be from 1:0.2 to 1:200, preferably 1:2 to 1:50, more preferably 1:15 to 1:25.

The pharmaceutical compositions according to the invention may comprise for example 850 mg of metformin and 80 mg of fenofibrate; 850 mg of metformin and 54 mg of fenofibrate; 500 mg of metformin and 80 mg of fenofibrate; 500 mg of metformin and 54 mg of fenofibrate; 500 mg of metformin and 40 mg of fenofibrate; or 1600 mg of metformin and 160 mg of fenofibrate, depending on whether the composition is to be taken once daily or more than once a day.

In one embodiment of the invention, the once-a-day or twice-a-day compositions are preferred.

To minimize the size of the combination metformin-fibrate in order to give to the pharmaceutical composition a size suitable for oral administration, the total drug content of the composition can be reduced whilst maintaining bioequivalence to the active compounds administered singly. For example, the composition of the invention can contain 150 mg or 70 mg of fenofibrate with respectively 1400 or 700 mg of metformin and remain bio-equivalent to a composition containing 160 mg or 80 mg of fenofibrate and 1500 mg or 850 mg of metformin. Another example of composition according to the invention can be one that contains 71 mg of fenofibrate and 850 mg of metformin.

In another embodiment of the invention, the composition can be a slow release formulation with, for example, reduce amount of active substance. Different sustained release of metformin is described in several patents such as US patent n°6,475,521, US patent n° 5, 972, 389, EP patent n° 1,335,708.

In one preferred embodiment of the invention, when the composition is under the form of a tablet for once-a-day administration, the weight of the tablet can vary from 1000 mg to 3000 mg, more preferably from 1500 mg to 2500 mg and even more preferably from 1700 mg to 2200 mg.

In another preferred embodiment of the invention, when the composition is under the form of a tablet for twice-a-day administration, the weight of the tablet can vary from 750 mg to 1500 mg, more preferably from 1000 mg to 1400 mg and even more preferably from 1100 mg to 1300 mg.

In another preferred embodiment of the invention, when the composition is under the form of a tablet for administration three times a day, the weight of the tablet can vary from 500 mg to 1000 mg, more preferably from 600 mg to 900 mg and even more preferably from 700 mg to 800 mg.

In another preferred embodiment of the invention, when the composition is under the form of a tablet for administration four times a day the weight of the tablet can vary from 500 mg to 1000 mg, more preferably from 600 mg to 900 mg and even more preferably from 650 mg to 750 mg.

In one embodiment of the invention, the pharmaceutical composition of the invention comprises a fibrate and metformin in a total amount of from about 50% to 99% by weight, preferably from about 60% to 98% by weight, more preferably from about 70% to 95% by weight, and even more preferably from 74 to 90% by weight based on the total weight of the composition.

The amount of excipients of the composition is in the range of from about 1 to about 50% by weight, preferably from about 2 to about 40%, more preferably from about 5 to about 30% by weight, and even more preferably from about 10% to about 26% by weight, based on the total weight of the composition.

The invention provides compositions wherein the pharmacokinetic profiles of the fibrate and of metformin are not substantially affected when administered to a human, in the sense that there is no substantial difference in the quantity of drug absorbed or the rate of drug absorption when the compositions of the invention are administered as compared to separate co-administration of each component.

The invention also encompasses a composition as defined above in which administration of the composition is bioequivalent to co-prescription of a composition containing either fibrate or metformin. "Bioequivalency" is established by a 90% Confidence Interval (CI) of between 0.80 and 1.25 for both Cₘₐₓ and AUC under USFDA regulatory guidelines, or a 90% CI for AUC of between 0.80 to 1.25 and a 90% CI for Cₘₐₓ of between 0.70 to 1.43 under the European EMEA regulatory guidelines.

In one embodiment of the invention, the compositions of the invention present a specific dissolution profile. Rapid dissolution of an administered active agent is preferable, as faster dissolution generally leads to faster onset of action and greater bioavailability. To improve the dissolution profile and bioavailability of fibrates, and in particular fenofibrate, it would be useful to increase the drug's dissolution so that it could attain a level close to 100%.

The compositions of the invention preferably have a dissolution profile in which within about 5 minutes at least about 10% of the fibrate, preferably fenofibrate, is dissolved. In another embodiment of the invention, preferably at least about 20% of the fibrate, preferably fenofibrate, is dissolved within about 5 minutes. In yet another embodiment of the invention, preferably at least about 20% of the fibrate, preferably fenofibrate, is dissolved within about 10 minutes. In another embodiment of the invention, at least about 50% of the fibrate, preferably fenofibrate, is dissolved within about 20 minutes. In another embodiment of the invention, at least about 70% of the fibrate, preferably fenofibrate, is dissolved within about 30 minutes.

Dissolution is generally measured in a medium which is discriminating. Such a dissolution medium will produce two very different dissolution curves for two products having very different dissolution profiles in gastric juices; *i.e.*, the dissolution medium is predictive of *in vivo* dissolution of a composition. An exemplary dissolution medium is an aqueous medium containing the surfactant sodium lauryl sulfate at 0.025 M. The determination of the amount dissolved can be carried out by spectrophotometry. The rotating blade method (75 rpm) according to the European Pharmacopoeia can be used to measure dissolution.

The pharmaceutical compositions of the present invention can be used for the treatment of non-insulin dependent diabetes mellitus (or type 2 diabetes), dyslipidemia (optionally associated with impaired glucose tolerance), hyperlipidemia, hypercholesterolemia or related conditions, for the prevention of cardiovascular events, coronary heart disease and peripheral vascular disease (including symptomatic carotid artery disease), for the treatment and prevention of metabolic syndrome and for the treatment or prevention of any illness in which a treatment with a fibrate and metformin is desirable, such as obesity.

The pharmaceutical compositions of the invention can also be used as adjunctive therapy to diet for the reduction of LDL-C, total-C, triglycerides, and Apo B in adult patients with primary hypercholesterolemia or mixed dyslipidemia (Fredrickson Types IIa and IIb). These compositions can also be used as adjunctive therapy to diet for treatment of adult patients with hypertriglyceridemia (Fredrickson Types IV and V hyperlipidemia). Markedly elevated levels of serum triglycerides (*e.g.,* > 2000 mg/dL) may in fact increase the risk of developing pancreatitis.

Accordingly, the invention also provides a method of treating or preventing the above-mentioned diseases, disorders or events, which comprises administering to a subject in need thereof the pharmaceutical composition as defined above.

The composition of the invention may additionally comprise, or be administered in combination with, one or more active substances selected from the group consisting of PPARγ activators, HMG CoA reductase inhibitors and antihypertensives.

Examples of PPARγ activators include, but are not limited to, thiazolidinedione compounds, such as rosiglitazone, pioglitazone, ciglitazone, englitazone, darglitazone and analogues and derivatives and pharmaceutically acceptable salts thereof.

Examples of HMG CoA reductase inhibitors (or statins) include, but are not limited to, lovastatin; pravastatin; simavastatin (Zocor®); velostatin; atorvastatin (Lipitor®) and other 6-[2-(substituted-pyrrol-1-yl)alkyl]pyran-2-ones and derivatives, as disclosed in U.S. Patent No. 4,647,576); fluvastatin (Lescol® ); fluindostatin (Sandoz XU-62-320); pyrazole analogs of mevalonolactone derivatives, as disclosed in WO 86/03488; rivastatin and other pyridyldihydroxyheptenoic acids, as disclosed in European Patent 491226A; dichloroacetate; imidazole analogs of mevalonolactone, as disclosed in PCT application WO 86/07054; 3-carboxy-2-hydroxy-propane-phosphonic acid derivatives, as disclosed in French Patent No. 2,596,393; 2,3-di-substituted pyrrole, furan, and thiophene derivatives, as disclosed in European Patent Application No. 0221025; naphthyl analogs of mevalonolactone, as disclosed in U.S. Patent No. 4,686,237; octahydronaphthalenes, such as those disclosed in U.S. Patent No. 4,499,289; keto analogs of mevinolin (lovastatin), as disclosed in European Patent Application No. 0,142,146 A2; phosphinic acid compounds; as well as other HMG CoA reductase inhibitors.

Examples of antihypertensives include, but are not limited to, diuretics ("water pills"), beta blockers, alpha blockers, alpha-beta blockers, sympathetic nerve inhibitors, angiotensin converting enzyme (ACE) inhibitors, calcium channel blockers, angiotensin receptor blockers.

The present invention also relates to processes for preparing granulates as defined above comprising particles of metformin surrounded by particles of a fibrate.

Preferred processes according to the present invention enable the production of granulates, comprising particles of metformin which are either isolated or agglomerated, and particles of a fibrate, adhering to said metformin particles. These processes include fluid bed granulation process in which an aqueous dispersion of fibrate is sprayed onto a fluidized bed of metformin, high shear granulation process and "one-pot" granulation process.

In a preferred embodiment according to the present invention, the process for preparing granulates is a fluid bed granulation process comprising the steps of:
a) preparing an aqueous dispersion of the fibrate, preferably in the presence of at least one binder and/or surface stabilizer;
b) spraying the resulting dispersion onto a fluidized bed of metformin, whereby granulates are obtained;
c) drying the resulting granulates.

According to an embodiment of this process, the dispersion prepared in step a) can further comprise one or more additives e.g. a controlled release barrier, a surfactant (or emulsifier) and/or a plasticizer.

According to another embodiment of this process, the dispersion in step a) is prepared from a nanoparticulate dispersion of fibrate, preferably fenofibrate, that can be obtained as described for example in US patent application 2003/0224058.

In another preferred embodiment, the process for preparing granulates is a high shear granulation process comprising the steps of:
a) subjecting to high-shear a mixture of metformin and the fibrate, preferably in the presence of at least one binder and/or surface stabilizer;
b) adding water to the high-sheared mixture whereby granulates are obtained;
c) drying the resulting granulates in a fluid bed dryer.

According to an embodiment of this process, a controlled release barrier is added in step a) to the mixture of metformin and fibrate, and a surfactant (emulsifier) and/or a plasticizer are further added in step b).

In yet another preferred embodiment, the process for preparing granulates is a "one-pot" granulation process comprising the steps of:
a) subjecting to high-shear a mixture of metformin and the fibrate, preferably in the presence of at least one binder and/or surface stabilizer;
b) adding water to the high-sheared mixture whereby granulates are obtained;
c) drying the resulting granulates in a one-pot system.

The drying step in the 'one pot' system is carried out by passing dry gas through the granulate bed, and applying heat thereto e.g. via an external jacket, by microwave radiation or by a combination of two or more of these methods.

Surprisingly, it has been found that the both the high shear and "one-pot" granulation methods enable compositions to be produced analogous to those produced by fluidised bed granulation method. The use of high shear granulation affords a process with relatively short processing times. The use of "one pot" granulation affords a process with relatively short processing times and a reduction in the number of processing steps required to produce the product.

In still another embodiment of the invention, a pharmaceutical composition can be prepared by a process comprising a) adding suitable excipients and, optionally, one or more active substances selected from the group consisting of PPARγ activators, HMG CoA reductase inhibitors and antihypertensives, to the granulates as defined above and b) formulating the resulting mixture or blend into the desired composition.

Several exemplary tablet formulations of the invention are given below. These examples are not intended to limit the claims in any respect, but rather provide exemplary tablet formulations of the invention. Such exemplary tablets can also comprise a coating agent.

The tablets of these examples are suitable for oral administration, i.e. can be easily swallowed. The weight of the 850 mg/80 mg dosage form or 850 mg/71 mg controlled release dosage form of the examples is comprised between approximately 1080 and 1440 g.

In these examples, the total drug content is from about 60 to 90 % by weight, based on the total weight of the tablets.

Both the foregoing description and the following examples are exemplary and explanatory and are intended to provide further explanation of the invention as claimed. Other objects, advantages, and novel features will be readily apparent to those skilled in the art from the description and examples of the invention.

### Example 1: Manufacture of pharmaceutical composition by fluid bed granulation (process A)

A pharmaceutical composition comprising fenofibrate and metformin was prepared as follows:
1. Water, povidone and fenofibrate (co-micronized with sodium lauryl sulfate and having an average particle size of approximately 8 µm) are stirred together to form dispersion A.
2. Metformin (having an average particle size of between 125 µm and 250 µm) is placed in the bowl of a fluid bed granulator and fluidised with air at 60°C - 70°C.
3. Dispersion A is sprayed onto the fluidised bed of metformin to effect granulation.
4. The granules are dried
5. The granules are sieved through a 1 mm sieve
6. Microcrystalline cellulose, crospovidone, colloidal silicon dioxide and magnesium stearate are added to the granules and blended.
7. Optionally, the blend can be compressed into tablets. The tablets can be coated. The blend can also be put in capsules.

### Example 2: Manufacture of pharmaceutical composition by high shear granulation (process B)

A pharmaceutical composition comprising fenofibrate and metformin was prepared as follows:
1. Povidone, fenofibrate (co-micronized with sodium lauryl sulfate and having an average particle size of approximately 8 µm) and metformin (having an average particle size of between 125 µm and 250 µm) are stirred together and subjected to high shear.
2. Water is added to this mixture to effect granulation
3. The resulting granules are transferred to a fluid bed dryer and dried
4. The dried granules are sieved through a 1 mm sieve
5. Microcrystalline cellulose, crospovidone, colloidal silicon dioxide and magnesium stearate are added to the granules and blended.
6. Optionally, the blend can be compressed into tablets. These tablets can be coated.

### Example 3: Manufacture of combination by means of 'one-pot' granulation (process C)

A pharmaceutical composition comprising fenofibrate and metformin was prepared as follows:
1. Povidone, fenofibrate (co-micronized with sodium lauryl sulfate and having an average particle size of approximately 8 µm) and metformin (having an average particle size of between 125 µm and 250 µm) are stirred together and subjected to high shear.
2. Water is added to this mixture to effect granulation
3. The resulting granules are dried within the 'one pot' system by means of passing dry gas through the granule bed, applying heat via an external jacket, by microwave radiation or by a combination of two or more of these methods.
4. The granules are sieved through a 1 mm sieve
5. Microcrystalline cellulose, crospovidone, colloidal silicon dioxide and magnesium stearate are added to the granules and blended.
6. Optionally, the blend can be compressed into tablets. These tablets can be coated.

### Example 4: Composition A

A tablet having the following composition was prepared according to process A:

| **Component** | **Per tablet (mg)** |
|---|---|
| Fenofibrate | 80 |
| Metformin | 850.00 |
| Sodium Lauryl Sulfate | 2.72 |
| Povidone | 44.84 |
| Crospovidone | 62.27 |
| Microcrystalline cellulose | 186.80 |
| Colloidal Silicon Dioxide | 12.45 |
| Magnesium stearate | 6.23 |
| Total | 1245.30 |

### Example 5: Composition B

A tablet having the following composition was prepared according to process B:

| **Component** | **Per tablet (mg)** |
|---|---|
| Fenofibrate | 80 |
| Metformin | 850.00 |
| Sodium Lauryl Sulfate | 2.72 |
| Povidone | 44.84 |
| Crospovidone | 62.27 |
| Microcrystalline cellulose | 186.80 |
| Colloidal Silicon Dioxide | 12.45 |
| Magnesium stearate | 6.23 |
| Total | 1245.30 |

### Example 6: Composition C

A tablet having the following composition was prepared according to process B:

| **Component** | **Per tablet (mg)** |
|---|---|
| Fenofibrate | 80.00 |
| Metformin | 850.00 |
| Sodium Lauryl Sulfate | 2.72 |
| Povidone | 28.76 |
| Crospovidone | 61.24 |
| Microcrystalline cellulose | 183.72 |
| Colloidal Silicon Dioxide | 12.25 |
| Magnesium stearate | 6.12 |
| Total | 1224.82 |

### Example 7: Composition D

A tablet having the following composition was prepared according to process B:

| **Component** | **Per tablet (mg)** |
|---|---|
| Fenofibrate | 80.00 |
| Metformin | 850.00 |
| Sodium Lauryl Sulfate | 65.22 |
| Povidone | 28.76 |
| Crospovidone | 65.22 |
| Microcrystalline cellulose | 195.67 |
| Colloidal Silicon Dioxide | 13.04 |
| Magnesium stearate | 6.52 |
| Total | 1304.44 |

The release profiles of these compositions (Composition A, B, C and D) versus fenofibrate monotherapy (80 mg) and versus metformin monotherapy (850 mg) were measured. The fenofibrate and metformin bioequivalence was also analysed.

The results are reported in the tables below, where the following abbreviations are used:
AUC: area under the drug concentration time curve
Cmax: maximal concentration (µg / ml)
CI : Confidence Interval

### Example 8: Fenofibrate bioequivalence analysis

| Composition | Process | AUC (90% CI) | Ratio AUC | Cmax (90% CI) | Ratio Cmax |
|---|---|---|---|---|---|
| A | A | 0.922-1.064 | 0.990 | 0.906-1.141 | 1.017 |
| B | B | 0.886-1.023 | 0.952 | 0.845-1.064 | 0.948 |
| C | B | 0.971-1.077 | 1.023 | 1.003-1.206 | 1.100 |
| D | B | 0.933-1.035 | 0.983 | 0.994-1.196 | 1.090 |

The results presented are derived from a comparison of the test product to 80 mg fenofibrate reference monotherapy.

### Example 9: Metformin bioequivalence analysis

| Composition | Process | AUC (90% CI) | Ratio AUC | Cmax (90% CI) | Ratio Cmax |
|---|---|---|---|---|---|
| A | A | 0.982-1.074 | 1.027 | 0.945-1.074 | 1.007 |
| B | B | 0.963-1.053 | 1.007 | 0.967-1.099 | 1.031 |
| C | B | 0.985-1.160 | 1.068 | 0.994-1.192 | 1.089 |
| D | B | 0.956-1.126 | 1.037 | 0.984-1.180 | 1.077 |

The results presented are derived from a comparison of the test product to 850 mg metformin reference monotherapy.

### Example 10: Dissolution profile

The purpose of this example was to evaluate the dissolution of a composition according to the invention.

The dissolution of metformin and fenofibrate tablets with formulations as detailed in examples 4-7 and prepared as detailed in examples 1 and 2 was tested in a dissolution medium.

The dissolution medium employed was an aqueous medium containing the surfactant sodium lauryl sulfate at 0.025 M. Determination of the amount dissolved was carried out by HPLC, and the tests were repeated 12 times. The rotating blade method (European Pharmacopoeia) was used under the following conditions:
volume of media: 1000 ml;
media temperature: 37 °C;
blade rotation speed: 75 rpm;
samples taken: 5, 15, 30, 45 and 60 minutes

The dissolution data for all the batches manufactured for the compositions A, B, C and D as described above with 850mg metformin/80mg fenofibrate, are listed in the table below.

| Batch | % Fenofibrate dissolved at various time-points | | | | |
|---|---|---|---|---|---|
| | 5 minutes | 15 minutes | 30 minutes | 45 minutes | 60 minutes |
| Composition A | 37.7 | 81.2 | 93.2 | 95.3 | 96.6 |
| Composition B | 38.0 | 81.8 | 91.8 | 93.2 | 93.9 |
| Composition C | 64.6 | 87.8 | 94.2 | 96.0 | 96.8 |
| Composition D | 39.5 | 69.9 | 79.2 | 85.3 | 86.9 |

The metformin dissolution profiles have not been reported here. For all batches, 100% dissolution of metformin is achieved by the 15 minute time-point. At 5 minutes, the metformin dissolution ranges from 56.9% to 100%.

U.S. Patent No. 6,277,405, for "Fenofibrate Pharmaceutical Composition Having High Bioavailability and Method for Preparing It", provides a micronized fenofibrate composition which has a dissolution profile of at least 10% in 5 min, 20% in 10 min, 50% in 20 min, and 75% in 30 min.

The above results show that the compositions of the invention exhibit a fast dissolution profile, at least as fast as that of the composition of US 6,277,405.

As shown in Example 8 and 9, the pharmacokinetic parameters of the fibrate and metformin of the compositions of the invention are the same as those obtained when the metformin or fibrate is administered as a single composition, to a human. Specifically, there was no substantial difference in the rate or quantity of drug absorption when the composition was administered versus co-administration. Thus, the compositions of the invention, preferably metformin and fenofibrate compositions, permit to substantially present the same pharmacokinetics of fibrate and of metformin when administered as a single product.

### Example 11: Composition E.

A tablet having the following composition was prepared according to the process detailed below:

| **Component** | **Per tablet (mg)** |
|---|---|
| Fenofibrate nanoparticles | 80.00 |
| Metformin | 850.00 |
| Docusate sodium | 1.59 |
| HPMC | 16.00 |
| Sodium Lauryl Sulfate | 5.61 |
| Sucrose | 80.00 |
| Crospovidone | 11.11 |
| Microcrystalline cellulose | 33.32 |
| Colloidal Silicon Dioxide | 2.22 |
| Magnesium stearate | 1.11 |
| Total | 1080.95 |

The tablets were manufactured according to process A outlined in example 1 with the exception that dispersion A is prepared by mixing together water, sucrose and a nanoparticulate dispersion of fenofibrate that can be obtained for example as described in US patent application 2003/0224058, the content of which is incorporated by reference.

### Example 12: Controlled release composition F.

A tablet having the following composition was prepared according to the process detailed below:

| **Component** | **Per tablet (mg)** |
|---|---|
| Fenofibrate | 80.00 |
| Metformin | 850.00 |
| Sodium Lauryl Sulfate | 65.22 |
| Povidone | 28.76 |
| Polymethacrylate | 125 |
| Mono / di glycerides | 7.5 |
| Polysorbate 80 | 3 |
| Crospovidone | 65.22 |
| Microcrystalline cellulose | 195.67 |
| Colloidal Silicon Dioxide | 13.04 |
| Magnesium stearate | 6.52 |
| Total | 1439.93 |

The tablets were manufactured according to process A outlined in example 1 with the exception that dispersion A is prepared by mixing water, povidone, fenofibrate, polymethacrylate, mono/di glycerides and polysorbate 80 together.

In this example the polymethacrylate functions as a controlled release barrier. The polysorbate 80 serves to emulsify the mono / di-glycerides which, in turn, serve to plasticize the polymethacrylate.

### Example 13: Controlled release composition G.

A tablet having the following composition was prepared according to the process outlined in example 12.

| **Component** | **Per tablet (mg)** |
|---|---|
| Fenofibric acid | 71.00 |
| Metformin | 850.00 |
| Sodium Lauryl Sulfate | 65.22 |
| Povidone | 28.76 |
| Polymethacrylate | 125 |
| Mono / di glycerides | 7.5 |
| Polysorbate 80 | 3 |
| Crospovidone | 65.22 |
| Microcrystalline cellulose | 195.67 |
| Colloidal Silicon Dioxide | 13.04 |
| Magnesium stearate | 6.52 |
| Total | 1430.93 |

### Example 14: Controlled release composition H.

A tablet having the following composition was prepared according to the process detailed below:

| **Component** | **Per tablet (mg)** |
|---|---|
| Fenofibric acid | 71.00 |
| Metformin | 850.00 |
| Sodium Lauryl Sulfate | 65.22 |
| Povidone | 28.76 |
| Polymethacrylate | 125 |
| Mono / di glycerides | 7.5 |
| Polysorbate 80 | 3 |
| Crospovidone | 65.22 |
| Microcrystalline cellulose | 195.67 |
| Colloidal Silicon Dioxide | 13.04 |
| Magnesium stearate | 6.52 |
| Total | 1430.93 |

This composition is manufactured according to process B outlined in example 2 with the exception that step 1 involves stirring together povidone, fenofibrate, metformin and dry polymethacrylate and subjecting them to high shear and step 2 involves adding a solution composed of mono/diglycerides and polysorbate 80 dissolved in water to the dry powder under conditions of high shear.

In this example the polymethacrylate functions as a controlled release barrier. The polysorbate 80 serves to emulsify the mono / di-glycerides which, in turn, serve to plasticize the polymethacrylate.

The composition according to example 4, 5, 6, 11 may be taken twice a day with meals.

Two doses of the controlled release composition according to example 12, 13, 14 may be taken once a day, preferably with the evening meal.

It will be apparent to those skilled in the art that various modifications and variations can be made in the methods and compositions of the present invention without departing from the spirit or scope of the invention. Thus, it is intended that the present invention cover the modifications and variations of this invention provided they come within the scope of the appended claims and their equivalents.

## Claims

1. Granulates comprising particles of metformin which are either isolated or agglomerated, and particles of a fibrate, adhering to said metformin particles.

2. The granulates of claim 1, wherein the fibrate is in a crystalline phase, an amorphous phase, a semi-crystalline phase, or a semi-amorphous phase.

3. The granulates of claim 1 or 2, wherein the fibrate is selected from the group consisting of gemfibrozil, fenofibrate, bezafibrate, clofibrate, ciprofibrate, beclobrate, binifibrate, ciplofibrate, clinofibrate, etofibrate, nicofibrate, pirifibrate, ronifibrate, simfibrate, or theofibrate, a fibric acid derivative or a pharmaceutically acceptable salt or ester of said fibric acid derivative.

4. The granulates of one of claims 1 to 3, wherein the fibrate is fenofibrate, fenofibric acid or a pharmaceutically acceptable salt or ester of fenofibric acid.

5. The granulates of one of claims 1 to 4, wherein the fibrate is fenofibrate.

6. The granulates of one of claims 1 to 5, wherein the fibrate is micronised or co-micronised.

7. The granulates of claim 6, wherein the fibrate is co-micronized with a surfactant.

8. The granulates of one of claim 1 to 7, wherein the particles of fibrate have an average size of less than about 20 µm, preferably of less than about 10 µm.

9. The granulates of one of claims 1 to 5, wherein the fibrate is in the form of nanoparticles having an average size of less than about 2000 nm, preferably of less than about 1500 nm, preferably of less than about 1000 nm, preferably of less than about 500 nm, and preferably of less than about 100 nm.

10. The granulates of one of claims 1 to 9, wherein metformin is in the form of the free base or one of its pharmaceutically acceptable salts.

11. The granulates of one of claims 1 to 10, wherein the particles of metformin and the particles of fibrate are bound together with a binder.

12. A pharmaceutical composition containing granulates of one of claims 1 to 11.

13. The pharmaceutical composition of claim 12, comprising from about 10 mg to about 3000 mg of fibrate.

14. The pharmaceutical composition of claim 13, comprising from about 10 to about 300 mg, preferably from about 40 to about 90 mg, of fibrate.

15. The pharmaceutical composition of one of claims 12 to 14, comprising from about 100 mg to about 3000 mg of metformin or a pharmaceutically acceptable salt thereof.

16. The pharmaceutical composition of claim 15, comprising from about 200 mg to about 2000 mg of metformin or a pharmaceutically acceptable salt thereof.

17. The pharmaceutical composition of one of claims 12 to 16, wherein the weight ratio of fibrate to metformin is from about 1:0.2 to about 1:200.

18. The pharmaceutical composition of one of claims 12 to 17, comprising 1600 mg of metformin and 160 mg of fenofibrate; 850 mg of metformin and 80 mg of fenofibrate; 850 mg of metformin and 54 mg of fenofibrate; 500 mg of metformin and 80 mg of fenofibrate; 500 mg of metformine and 54 mg of fenofibrate; or 500 mg of metformin and 40 mg of fenofibrate.

19. The pharmaceutical composition of one of claims 12 to 18, comprising:
• from about 50% to about 99% by weight, preferably from about 60% to about 98% by weight, preferably from about 70% to about 95% by weight, preferably from about 74% to about 90% by weight of fibrate and metformin; and
• from about 1% to about 50% by weight, preferably from about 2% to about 40% by weight, preferably from about 5% to about 30% by weight, preferably from about 10% to about 26% by weight of pharmaceutically acceptable excipients.

20. The pharmaceutical composition of one of claims 12 to 19, wherein the composition is formulated for oral, pulmonary, rectal, ophthalmic, colonic, parenteral, intracisternal, intravaginal, intraperitoneal, local, buccal, nasal, or topical administration.

21. The pharmaceutical composition of one of claims 12 to 19, which is a tablet.

22. The tablet of claim 21, weighing from about 500 to about 3000 mg.

23. The pharmaceutical composition of one of claims 12 to 19, which is a capsule.

24. The pharmaceutical of one of claims 12 to 23, in which at least about 10% of the fibrate is dissolved within about 5 minutes, at least about 20% of the fibrate is dissolved within about 10 minutes, at least about 50% of the fibrate is dissolved within about 20 minutes, and at least about 70% of the fibrate is dissolved within about 30 minutes, as measured using the rotating blade method at 75 rpm according to the European Pharmacopoeia, in a dissolution medium containing 0.025 M sodium lauryl sulfate.

25. The pharmaceutical composition of one of claims 12 to 23, further comprising one or more active substances selected from the group consisting of PPARγ activators, HMG CoA reductase inhibitors and antihypertensives.

26. A process for preparing granulates as defined in one of claims 1 to 11, which process comprises the steps of:
a) preparing an aqueous dispersion of the fibrate, preferably in the presence of at least one binder and/or surface stabilizer;
b) spraying the resulting dispersion onto a fluidized bed of metformin, whereby granulates are obtained;
c) drying the resulting granulates.

27. A process for preparing granulates as defined in one of claims 1 to 11, which process comprises the steps of:
a) subjecting to high-shear a mixture of metformin and the fibrate, preferably in the presence of at least one binder and/or surface stabilizer;
b) adding water to the high-sheared mixture whereby granulates are obtained;
c) drying the resulting granulates in a fluid bed dryer.

28. A process for preparing granulates as defined in one of claims 1 to 11, which process comprises the steps of:
a) subjecting to high-shear a mixture of metformin and the fibrate, preferably in the presence of at least one binder and/or surface stabilizer;
b) adding water to the high-sheared mixture whereby granulates are obtained;
c) drying the resulting granulates in a one-pot system.

29. A process for preparing a pharmaceutical composition as defined in one of claims 12 to 25, which comprises a) adding suitable excipients and, optionally, one or more active substances selected from the group consisting of PPARγ activators, HMG CoA reductase inhibitors and antihypertensives, to the granulates of one of claims 1 to 11; and b) formulating the resulting mixture into the desired composition.
